(19) Europäisches Patentamt
European Patent Office
Office européen des brevets

(11) EP 1 462 088 A1

(12) DEMANDE DE BREVET EUROPEEN

(43) Date de publication:
**29.09.2004 Bulletin 2004/40**

(51) Int Cl.[7]: **A61K 7/075**, A61K 7/06

(21) Numéro de dépôt: **04290563.8**

(22) Date de dépôt: **02.03.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **25.03.2003 FR 0303643**

(71) Demandeur: **L'OREAL**
**75008 Paris (FR)**

(72) Inventeurs:
- **Müller, Rainer**
**76344 Leopoldshafen (DE)**
- **Desenne, Patricia**
**92270 Bois Colombes (FR)**

(74) Mandataire: **Le Blainvaux Bellegarde, Françoise**
**L'OREAL - D.I.P.I.**
**25-29 Quai Aulagnier**
**92600 Asnières (FR)**

# (54) Composition de traitement des matières kératiniques comprenant un acide hydroxycarboxylique et un agent protecteur ou conditionneur

(57) L'invention concerne une composition de traitement des matières kératiniques, en particulier les cheveux, comprenant, dans un milieu physiologiquement et en particulier cosmétiquement acceptable, au moins un agent protecteur ou conditionneur, et au moins un acide hydroxycarboxylique particulier ou ses sels.

Ces associations permettent d'améliorer le dépôt de l'agent protecteur ou conditionneur des matières kératiniques ainsi que les propriétés cosmétiques.

EP 1 462 088 A1

## Description

L'invention concerne une composition de traitement des matières kératiniques, en particulier des fibres kératiniques humaines telles que les cheveux, comprenant, dans un milieu physiologiquement et en particulier cosmétiquement acceptable, au moins un agent protecteur ou conditionneur, et au moins un acide hydroxycarboxylique particulier ou ses sels.

Il est bien connu que les cheveux sont sensibilisés ou fragilisés à des degrés divers par l'action des agents atmosphériques et notamment de la lumière, l'eau et l'humidité ainsi que par l'action répétée des différents traitements capillaires tels que le lavage, les permanentes, le défrisage, la teinture, la décoloration. De nombreuses publications divulguent que la lumière naturelle détruit certains aminoacides des cheveux. Ces agressions altérant la fibre capillaire, elles en diminuent les propriétés mécaniques comme la résistance à la traction, la charge à la rupture et l'élasticité, ou leur résistance au gonflement dans un milieu aqueux. Les cheveux sont alors ternes, rêches et cassants. Les cheveux, contrairement à la peau, éclaircissent.

On sait également que notamment la lumière et les agents de lavage ont tendance à agresser la couleur naturelle des cheveux ainsi que la couleur artificielle des cheveux teints. La couleur des cheveux s'affaiblit peu à peu ou vire vers des nuances peu esthétiques ou indésirables.

On recherche depuis de nombreuses années, dans l'industrie cosmétique des substances permettant de protéger les cheveux des dégradations engendrées par les agressions atmosphériques, telles que la lumière et la chaleur et les traitements. En particulier, on recherche des produits protégeant la couleur des fibres kératiniques colorées naturellement ou teintes artificiellement et préservant ou renforçant les propriétés mécaniques intrinsèques des fibres kératiniques (la résistance à la traction, la charge à la rupture et l'élasticité, ou leur résistance au gonflement dans un milieu aqueux).

Pour lutter contre ces dégradations de la kératine des cheveux, on a déjà proposé d'utiliser certaines substances susceptibles de filtrer les radiations lumineuses, comme l'acide 2-hydroxy-4-méthoxy benzophénone-5-sulfonique ou ses sels (FR-A-2 627 085) ou l'acide 4-(2-oxo-3-bornylidène méthyl) benzène sulfonique ou ses sels (EP-A-329 032) ou encore la lactoferrine (FR-A-2 673 839).

Cependant, ces filtres lorsqu'ils sont efficaces ne le sont qu'à des concentrations importantes. Or, à ces concentrations, les cheveux traités avec ces filtres présentent un toucher rêche et chargé. De plus, le démêlage est extrêmement difficile.

On a déjà préconisé dans les compositions pour le lavage ou le soin des matières kératiniques telles que les cheveux l'utilisation d'agents conditionneurs, notamment des polymères cationiques ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe).

En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

En résumé, il s'avère que les compositions cosmétiques actuelles contenant des agents protecteurs ou conditionneurs, ne donnent pas complètement satisfaction.

Par ailleurs, les compositions cosmétiques contiennent généralement un agent complexant destiné à complexer les cations métalliques susceptibles de se trouver à l'état de traces dans ces compositions, ainsi que ceux pouvant être présents sur les cheveux et provenant de l'air ambiant, de l'eau avec laquelle ces derniers ont été lavés ou encore des shampoings ou autres produits capillaires avec lesquels ils ont été traités. Il est, en effet, très important de neutraliser ces cations métalliques, dans la mesure où ils sont susceptibles de catalyser les réactions d'oxydation des fibres capillaires et ce, de façon non contrôlée, ce qui peut se traduire par des effets indésirables sévères tels qu'une cassure des cheveux ou une brûlure du cuir chevelu.

Actuellement, les agents complexants les plus couramment utilisés dans les compositions oxydantes pour la coloration, la décoloration ou la déformation permanente de fibres kératiniques sont l'acide éthylènediamine tétraacétique (EDTA) et ses dérivés comme l'acide diéthylènetriamine pentaacétique (DPTA), généralement dans des proportions pondérales de l'ordre de 0,1 à 1%.

Toutefois, dans le cadre de ses travaux, la Demanderesse a constaté que l'EDTA et ses dérivés présentent, dans ce type de compositions, des propriétés complexantes insuffisantes. Ces constatations, qui sont corroborées par les

résultats obtenus par d'autres équipes de recherche, justifient de trouver de nouveaux agents complexants.

Un agent complexant destiné à entrer dans la constitution de compositions cosmétiques doit satisfaire à de nombreuses exigences. En effet, outre qu'il doit présenter un pouvoir complexant élevé vis-à-vis des métaux de manière à supprimer ou, à tout le moins, réduire le plus possible le risque d'une catalyse des réactions d'oxydation des fibres kératiniques par les traces métalliques susceptibles d'être présentes dans ces compositions et sur ces fibres, il doit être compatible, et notamment ne pas réagir, avec les autres constituants. Il doit également être stable en solution. Il doit aussi être d'une innocuité totale pour ces fibres et pour la peau, et notamment être dénué de tout caractère allergène.

La demanderesse a maintenant découvert que l'association d'un complexant particulier avec des agents protecteurs ou conditionneurs permet d'augmenter le dépôt de l'agent protecteur ou conditionneur des matières kératiniques et par la même d'augmenter la protection ou le conditionnement.

On observe notamment une amélioration des propriétés cosmétiques telles qu'en particulier la facilité de coiffage, le maintien, la nervosité et le volume des cheveux traités.

Toutes ces découvertes sont à la base la présente invention.

Ainsi, selon la présente invention, il est maintenant proposé de nouvelles compositions cosmétiques, comprenant, dans un milieu physiologiquement et en particulier cosmétiquement acceptable, au moins un agent protecteur ou conditionneur particulier des matières kératiniques et au moins un acide hydroxycarboxylique particulier ou ses sels.

L'invention a donc pour objet une composition de traitement des matières kératiniques, en particulier des fibres kératiniques humaines telles que les cheveux, comprenant dans un milieu physiologiquement et en particulier cosmétiquement acceptable au moins un acide hydroxycarboxylique ou ses sels répondant à la formule générale (1) suivante :

$$R\text{-}(CHOH)_4\text{-}CO_2X \qquad (I)$$

dans laquelle :

- R représente un groupe $CO_2X$, et
- X représente un atome d'hydrogène ou un cation monovalent ou divalent issu d'un métal de transition, d'un métal alcalin ou alcalinoterreux, d'une amine organique ou d'un ion ammonium,

et au moins un agent protecteur et/ou un agent conditionneur, ledit agent conditionneur étant choisi parmi les huiles de synthèse, les huiles végétales, les huiles fluorées ou perfluorées, les cires naturelles ou synthétiques, les silicones, les polymères cationiques non polysaccharides, les composés de type céramide, les tensioactifs cationiques, les amines grasses, les acides gras et leurs dérivés, ainsi que les mélanges de ces différents composés.

Un autre objet de l'invention concerne l'utilisation d'au moins un acide hydroxycarboxylique particulier ou ses sels dans, ou pour la fabrication d'une composition cosmétique comprenant un agent protecteur ou conditionneur des matières kératiniques.

L'invention a également pour objet l'utilisation d'au moins un acide hydroxycarboxylique particulier ou ses sels dans une composition comprenant un agent protecteur ou conditionneur des matières kératiniques pour augmenter l'efficacité de cet agent protecteur ou conditionneur des matières kératiniques.

La présente invention a aussi pour objet l'utilisation d'au moins un acide hydroxycarboxylique particulier ou ses sels dans une composition comprenant un agent protecteur ou conditionneur des matières kératiniques pour améliorer le dépôt et/ou la fixation dudit agent protecteur ou conditionneur sur les matières kératiniques.

Les différents objets de l'invention vont maintenant être détaillés. L'ensemble des significations et définitions des composés utilisés dans la présente invention données ci-dessous sont valables pour l'ensemble des objets de l'invention.

Les acides hydroxycarboxyliques particuliers ou ses sels répondent à la formule générale (1) suivante :

$$R\text{-}(CHOH)_4\text{-}CO_2X \qquad (I)$$

dans laquelle :

R représente un groupe $CO_2X$ et
X représente un atome d'hydrogène ou un cation monovalent ou divalent issu d'un métal de transition, d'un métal alcalin ou alcalinoterreux, d'une amine organique ou d'un ion ammonium.

Ainsi, les agents complexants utilisés dans le cadre de l'invention correspondent à des acides hydroxycarboxyliques et aux carboxylates correspondants.
La formule (1) comprenant 4 groupes d'atomes H-C-OH chiraux, il va de soi que cette formule englobe tous les énantiomères et tous les diastéréoisomères des composés susceptibles de répondre à cette formule.
Conformément à l'invention, le cation monovalent ou divalent est, de préférence, choisi dans le groupe constitué par les cations de métaux alcalins, les cations de métaux alcalino-terreux et les cations divalents de métaux de transition. A titre d'exemples de cations de métaux alcalins, on peut notamment citer le sodium ($Na^+$) et le potassium ($K^+$), tandis qu'à titre d'exemples de cations de métaux alcalinoterreux, on peut notamment citer le calcium ($Ca^{2+}$) et le magnésium ($Mg^{2+}$).

Au sens de la présente invention, on entend par "métal de transition", un métal comportant une sous-couche d incomplète, plus particulièrement à l'état d'oxydation II, tel que le cobalt ($Co^{2+}$), le fer ($Fe^{2+}$), le manganèse ($Mn^{2+}$), le zinc ($Zn^{2+}$) et le cuivre ($Cu^{2+}$). Le ou les composés de formule (1) sont, de préférence, choisis dans le groupe constitué par l'acide mucique ($C_6H_{10}O_8$) - encore connu sous le nom d'acide galactarique -, l'acide glucarique ($C_6H_{10}O_8$), l'acide mannarique ($C_6H_{10}O_8$), leurs sels de métaux alcalins, leurs sels de métaux alcalino-terreux, leurs sels de métaux de transition, et leurs mélanges comme, par exemple, des mélanges d'acide mucique et de mucate de sodium ($C_6H_8O_8Na_2$).

De manière particulièrement préférée, le ou les composés de formule (1) est l'acide mucique.

De préférence, le ou les composés de formule (1) représentent de 0,001 à 10% en poids et, mieux encore, de 0,001 à 5% en poids du poids total de la composition.

Les agents protecteurs des matières kératiniques peuvent être tout agent actif utile pour prévenir ou limiter les dégradations dues aux agressions physiques ou chimiques.

Ainsi, l'agent protecteur des matières kératiniques peut être choisi parmi les filtres UV organiques hydrosolubles, liposolubles ou insolubles dans l'eau, les agents antiradicalaires, les agents antioxydants, les vitamines, les provitamines.

Les filtres UV organiques (systèmes filtrant les radiations UV) sont notamment choisis parmi les filtres hydrosolubles ou liposolubles, siliconés ou non siliconés et les nanoparticules d'oxydes minéraux dont la surface a éventuellement été traitée pour la rendre hydrophile ou hydrophobe.

Les filtres UV organiques hydrosolubles peuvent être choisis parmi par exemple, l'acide para-aminobenzoïque et ses sels, l'acide anthranilique et ses sels, l'acide salicylique et ses sels, l'acide p-hydroxycinnamique et ses sels, les dérivés sulfoniques de benz-x-azole (benzothioazoles, benzimidazoles, benzoxazoles) et leurs sels, les dérivés sulfoniques de la benzophénone et leurs sels, les dérivés sulfoniques de benzylidène camphre et leurs sels, les dérivés de benzylidène camphre substitués par une amine quaternaire et leurs sels, les dérivés des acides phtalydène-camphosulfoniques et leurs sels, les dérivés sulfoniques de benzotriazole et leurs mélanges.

On peut également utiliser des polymères hydrophiles présentant, en outre et de par leur nature chimique, des propriétés de photoprotection contre le rayonnement UV. On peut citer les polymères comportant des groupements benzylidène camphre et/ou benzotriazole, substitués par des groupements sulfoniques ou ammonium quaternaires.

Comme filtres UV organiques liposolubles (ou lipophiles) convenant à une mise en oeuvre dans la présente invention, on peut notamment citer : les dérivés d'acide p-aminobenzoïque, tels que les esters ou amides d'acide p-aminobenzoïque ; les dérivés d'acide salicylique tels que les esters; les dérivés de benzophénone ; les dérivés de dibenzoylméthane ; les dérivés de diphénylacrylates ; les dérivés de benzofurannes ; les filtres UV polymères contenant un ou plusieurs résidus silico-organiques ; les esters d'acide cinnamique ; les dérivés de camphre ; les dérivés de trianilino-s-triazine ; l'ester éthylique d'acide urocanique ; les benzotriazoles ; les dérivés d'hydroxyphényltriazine ; les bis-résorcinol-dialkylaminotriazine ; et leurs mélanges.

Le filtre UV liposoluble (ou lipophile) selon l'invention est de préférence choisi parmi : l'octyl salicylate ; le 4-tertiobutyl 4'-méthoxydibenzoylméthane (PARSOL 1789 de GIVAUDAN); l'octocrylène ; le 4-méthoxy cinnamate de 2-éthylhexyl (PARSOL MCX) et le composé de formule (V) suivante, ou 2-(2H-benzotriazole-2-yl)-4-méthyl-6-[2-méthyl-3-[1,3,3,3-tétraméthyl-1-[(triméthylsilyl)oxy] disiloxanyl]propynyl]phénol, décrit dans la demande de brevet EP-A-0 392 883 :

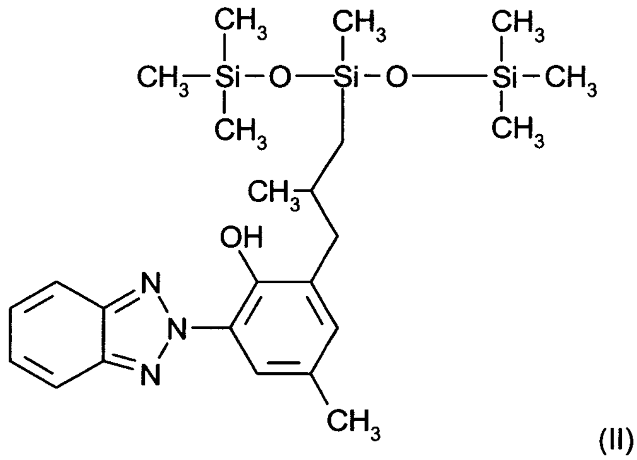

(II)

D'autres filtres UV particulièrement préférés selon l'invention sont les dérivés de benzophénones tels que l'UVINUL MS 40 (Acide 2-hydroxy 4-méthoxybenzophénone-5-sulfonique) et l'UVINUL M40 (2-hydroxy-4-méthoxybenzophénone) commercialisés par BASF, les dérivés de benzalmalonates tels que le PARSOL SLX (poly diméthyl/méthyl (3(4-(2,2-bis-éthoxycarbonylvinyl)-phénoxy)-propényl) siloxane) commercialisé par GIVAUDAN-ROURE, les dérivés de benzylidènecamphre tels que le MEXORYL SX (acide b-b'camphosulfonique [1-4 divinylbenzène]) fabriqué par la société CHIMEX, les dérivés de benzimidazole tels que l'EUSOLEX 232 (acide 2-phényl-benzimidazol-5-sulfonique) commercialisé par MERCK.

Les oxydes minéraux peuvent être choisis parmi les oxydes de titane, les oxydes de zinc et les oxydes de cérium.

Les agents antioxydants et/ou antiradicalaires sont notamment choisis parmi les phénols tels que le BHA (tert-butyl-4-hydroxyanisole), le BHT (2,6-di-tert-butyl-p-crésol), le TBHQ (tertiobutylhydroquinone), les polyphénols tels que les oligomères proanthocyanidoliques et les flavonoïdes, les amines encombrées connues sous le vocable générique de HALS (Hindered Amine Light Stabilizer) telles que la tétraaminopipéridine, l'acide érythorbique, les polyamines telles que la spermine, la cystéines, le glutathion, la superoxyde dismutase, la lactoferrine.

Les vitamines sont notamment choisies parmi l'acide ascorbique, la vitamine E, l'acétate de vitamine E, les vitamines B telles que les vitamines B3 et B5, la vitamine PP, la vitamine A et ses dérivés.

Les provitamines sont notamment choisies parmi le panthénol et le rétinol.

Selon l'invention, le ou les agents protecteurs des matières kératiniques peuvent représenter de 0,001 % à 20 % en poids, de préférence de 0,01 % à 10% en poids et plus particulièrement de 0,1 à 5% en poids par rapport au poids total de la composition finale.

Dans le cadre de la présente demande, on entend par agent conditionneur tout agent ayant pour fonction l'amélioration des propriétés cosmétiques des cheveux, en particulier la douceur, le démêlage, le toucher, le lissage, l'électricité statique.

Les agents de conditionnement peuvent se présenter sous forme liquide, semi-solide ou solide tels que par exemple des huiles, des cires ou des gommes.

Selon l'invention, les agents conditionneurs peuvent être choisis parmi les huiles de synthèses telles que les poly-oléfines, les huiles végétales, les huiles fluorées ou perfluorées, les cires naturelles ou synthétiques, les silicones, les polymères cationiques non polysaccharides, les composés de type céramide, les tensioactifs cationiques, les amines grasses, les acides gras et leurs dérivés ainsi que les mélanges de ces différents composés.

Les agents conditionneurs préférés selon l'invention sont les polymères cationiques et les silicones.

Les huiles de synthèse sont notamment les polyoléfines en particulier les poly-$\alpha$-oléfines et plus particulièrement :

- de type polybutène, hydrogéné ou non, et de préférence polyisobutène, hydrogéné ou non.

On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.

A titre d'exemples de poly-$\alpha$-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3)

par la Société ICI (n désignant le degré de polymérisation),

- de type polydécène, hydrogéné ou non.

De tels produits sont vendus par exemple sous les dénominations ETHYLFLO par la société ETHYL CORP., et d'ARLAMOL PAO par la société ICI.

Les huiles animales ou végétales sont choisies préférentiellement dans le groupe formé par les huiles de toumesol, de maïs, de soja, d'avocat, de jojoba, de courge, de pépins de raisin, de sésame, de noisette, les huiles de poisson, le tricaprocaprylate de glycérol, ou les huiles végétales ou animales de formule $R_9COOR_{10}$ dans laquelle $R_9$ représente le reste d'un acide gras supérieur comportant de 7 à 29 atomes de carbone et $R_{10}$ représente une chaîne hydrocarbonée linéaire ou ramifiée contenant de 3 à 30 atomes de carbone en particulier alkyle ou alkényle, par exemple, l'huile de Purcellin ou la cire liquide de jojoba ;

On peut également utiliser les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre 1983, pp. 30-33.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les polymères cationiques non saccharidiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519863.

Par polymères non saccharides, on entend les polymères ne contenant pas de liaison glycoside entre des monosaccharides.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant au moins un des motifs de formules suivantes:

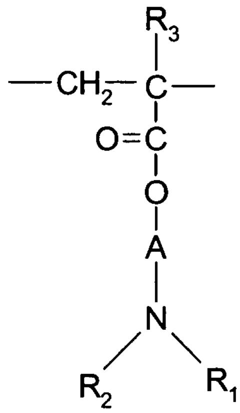 (III),

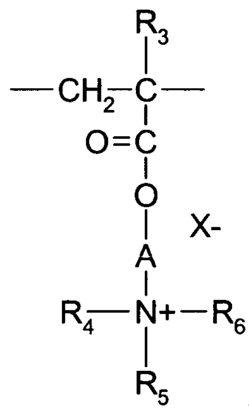 (IV)

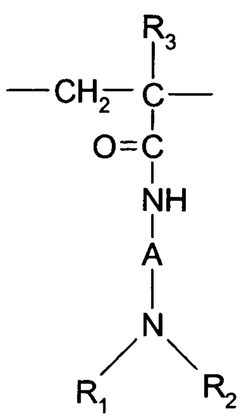 (V)

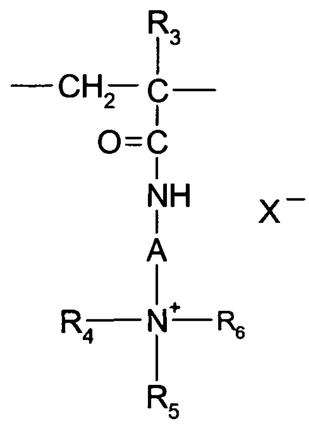 (VI)

dans lesquelles:

$R_1$ et $R_2$ , identiques ou différents, représentent hydrogène ou un groupe alkyle ayant de 1 à 6 atomes de carbone et de préférence méthyle ou éthyle;
$R_3$ , identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
X désigne un anion dérivé d'un acide minéral ou organique tel que un anion méthosulfate

ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.

Ainsi, parmi ces copolymères de la famille (1), on peut citer :

- les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société

CIBA GEIGY,

- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP.
- et les copolymères vinylpyrrolidone / méthacrylamide de diméthylaminopropyle quaternisé tel que le produit vendu sous la dénomination "GAFQUAT HS 100" par la société ISP.

(2) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quatemisation de ces polymères. De tels polymères sont notamment décrits dans les brevets français 2.162.025 et 2.280.361 ;

(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(4) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8: 1. De tels polymères sont notamment décrits dans les brevets américains 3.227.615 et 2.961.347.

Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(6) les cyclopolymères d'alkyl diallyl amine ou de dialkyl diallyl ammonium tels que les homopolymères ou copolymères comportant comme constituant principal de la chaîne des motifs répondant aux formules (VII) ou (VIII) :

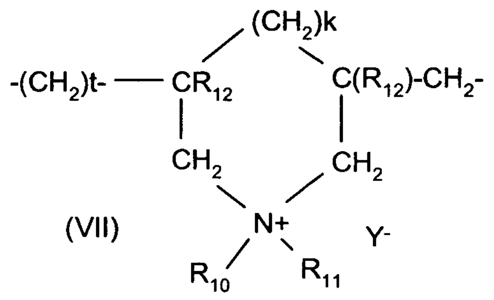

(VII)

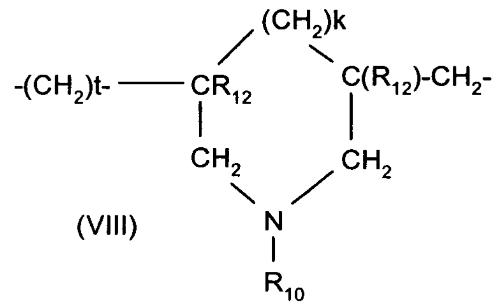

(VIII)

formules dans lesquelles k et t sont égaux à 0 ou 1, la somme k + t étant égale à 1 ; $R_{12}$ désigne un atome d'hydrogène ou un radical méthyle ; $R_{10}$ et $R_{11}$, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou $R_{10}$ et $R_{11}$ peuvent désigner conjointement avec l'atome d'azote auquel ils sont rattachés, des groupement hétérocycliques, tels que pipéridinyle ou morpholinyle ; Y- est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

$R_{10}$ et $R_{11}$, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "Merquat 100" par la société NALCO (et ses homologues de faibles masses moléculaires moyenne en poids) et les copolymères de chlorure de diallyldiméthylammonium et d'acrylamide commercialisés sous la dénomination "MERQUAT 550".

(7) le polymère de diammonium quaternaire contenant des motifs récurrents répondant à la formule :

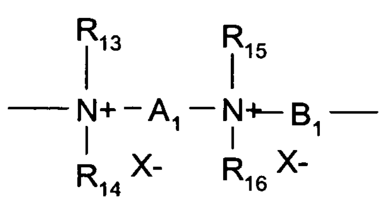

(IX)

formule (IX) dans laquelle :

$R_{13}$, $R_{14}$, $R_{15}$ et $R_{16}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote ou bien $R_{13}$, $R_{14}$, $R_{15}$ et $R_{16}$ représentent un radical alkyle en C1-C6 linéaire ou ramifié substitué par un groupement nitrile, ester, acyle, amide ou -CO-O-$R_{17}$-D ou -CO-NH-$R_{17}$-D où $R_{17}$ est un alkylène et D un groupement ammonium quaternaire ;

A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et

X- désigne un anion dérivé d'un acide minéral ou organique;

A1 , $R_{13}$ et $R_{15}$ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement $(CH_2)_n$-CO-D-OC-$(CH_2)$n-

n est un nombre entier variant de 2 à 20 environ

dans lequel D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)_x-CH_2-CH_2-$$

$$-[CH_2-CH(CH_3)-O]_y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH- ;

De préférence, X- est un anion tel que le chlorure ou le bromure.
Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule :

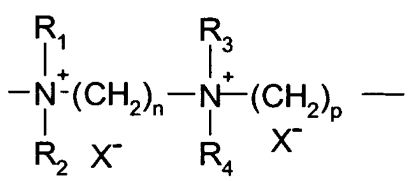
(a)

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
Un composé de formule (a) particulièrement préféré est celui pour lequel $R_1$, $R_2$, $R_3$ et $R_4$, représentent un radical méthyle et n = 3, p = 6 et X = Cl, dénommé Hexadimethrine chloride selon la nomenclature INCI (CTFA).

(8) les polymères de polyammonium quaternaires constitués de motifs de formule (X):

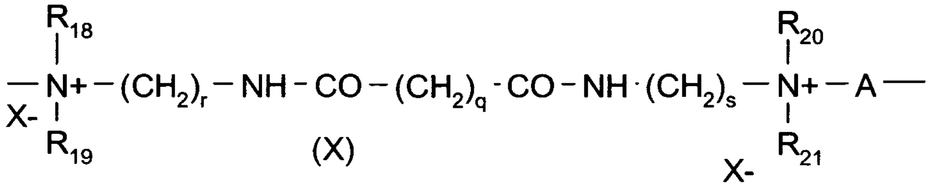
(X)

formule dans laquelle :

$R_{18}$, $R_{19}$, $R_{20}$ et $R_{21}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$,

où p est égal à 0 ou à un nombre entier compris entre 1 et 6, sous réserve que $R_{18}$, $R_{19}$, $R_{20}$ et $R_{21}$ ne

représentent pas simultanément un atome d'hydrogène,
r et s, identiques ou différents, sont des nombres entiers compris entre 1 et 6,
q est égal à 0 ou à un nombre entier compris entre 1 et 34,
X- désigne un anion tel qu'un halogènure,

A désigne un radical d'un dihalogénure ou représente de préférence $-CH_2-CH_2-O-CH_2-CH_2-$.
De tels composés sont notamment décrits dans la demande de brevet EP-A-122 324.
On peut par exemple citer parmi ceux-ci, les produits "Mirapol® A 15", "Mirapol® AD1", "Mirapol® AZ1" et "Mirapol® 175" vendus par la société Miranol.

(9) Les polymères quaternaires de vinylpyrrolidone et de vinylimidazole tels que par exemple les produits commercialisés sous les dénominations Luviquat® FC 905, FC 550 et FC 370 par la société B.A.S.F.

(10) Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/ chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.
Parmi tous les polymères cationiques susceptibles d'être utilisés dans le cadre de la présente invention, on préfère mettre en oeuvre les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole et leurs mélanges.
Les silicones utilisables conformément à l'invention sont en particulier des polyorganosiloxanes insolubles dans la composition et peuvent se présenter sous forme d'huiles, de cires, de résines ou de gommes.
Les organopolysiloxanes sont définis plus en détail dans l'ouvrage de Walter NOLL "Chemistry and Technology of Silicones" (1968) Académie Press. Elles peuvent être volatiles ou non volatiles.
Lorsqu'elles sont volatiles, les silicones sont plus particulièrement choisies parmi celles possédant un point d'ébullition compris entre 60° C et 260° C, et plus particulièrement encore parmi :

(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.

On peut également citer les cyclocopolymères du type diméthylsiloxanes/ méthylakylsiloxane, tel que la "SILICONE VOLATILE FZ 3109" commercialisée par la société UNION CARBIDE. de structure chimique :

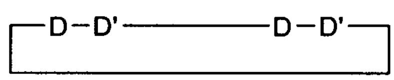

avec D : 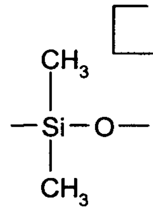 avec D' : 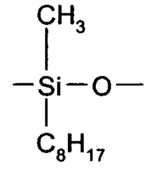

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;

(ii) les silicones volatiles linéaires ayant 2 à 9 atomes de silicium et possédant une viscosité inférieure ou égale à $5.10^{-6} m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de $5.10^{-6}$ à 2,5 $m^2/s$ à 25°C et de préférence $1.10^{-5}$ à 1 $m^2/s$. La viscosité des silicones est par exemple mesurée à 25°C selon la norme ASTM 445 Appendice C.

Parmi ces polyalkylsiloxanes, on peut citer à titre non limitatif les produits commerciaux suivants :

- les huiles SILBIONE des séries 47 et 70 047 ou les huiles MIRASIL commercialisées par RHODIA CHIMIE telles que par exemple l'huile 70 047 V 500 000 ;
- les huiles de la série MIRASIL commercialisées par la société RHODIA CHIMIE ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE .

Dans cette classe de polyalkylsiloxanes, on peut également citer les produits commercialisés sous les dénominations "ABIL WAX 9800 et 9801" par la société GOLDSCHMIDT qui sont des polyalkyl ($C_1$-$C_{20}$) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de $1.10^{-5}$ à $5.10^{-2} m^2/s$ à 25°C.

Parmi ces polyalkylarylsiloxanes on peut citer à titre d'exemple les produits commercialisés sous les dénominations suivantes :

. les huiles SILBIONE de la série 70 641 de RHODIA CHIMIE ;
. les huiles des séries RHODORSIL 70 633 et 763 de RHODIA CHIMIE ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :

- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

. les mélanges formés à partir d'un polydiméthylsiloxane hydroxylé en bout de chaîne (dénommé diméthiconol selon

la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;

- . les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- . les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6} m^2/s$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :

$R_2SiO_{2/2}$, $R_3SiO_{1/2}$, $RSiO_{3/2}$ et $SiO_{4/2}$ dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en $C_1$-$C_4$, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl ($C_{12}$) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (XI) :

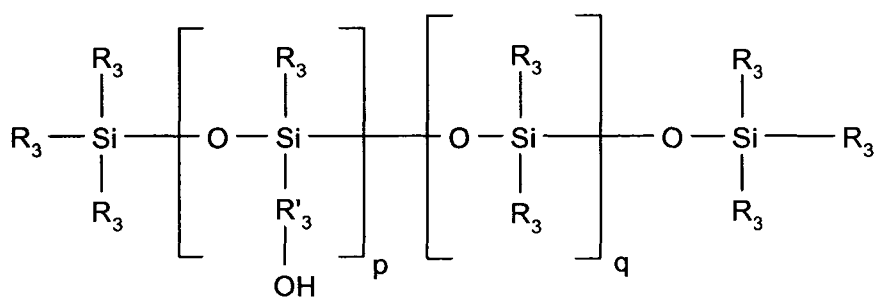

(XI)

dans laquelle les radicaux $R_3$ identiques ou différents sont choisis parmi les radicaux méthyle et phényle ; au moins 60 % en mole des radicaux $R_3$ désignant méthyle ; le radical $R'_3$ est un chaînon alkylène divalent hydrocarboné en $C_2$-$C_{18}$; p est compris entre 1 et 30 inclus ; q est compris entre 1 et 150 inclus ;

- des groupements acyloxyalkyle tels que par exemple les polyorganosiloxanes décrits dans le brevet US-A-4957732 et répondant à la formule (XII) :

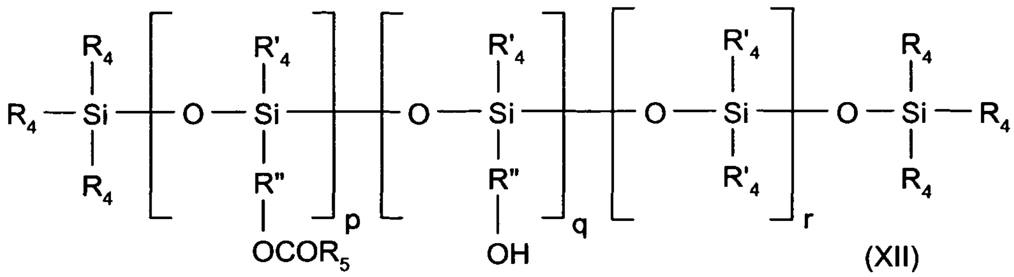

(XII)

dans laquelle :

$R_4$ désigne un groupement méthyle, phényle, $-OCOR_5$, hydroxyle, un seul des radicaux $R_4$ par atome de silicium pouvant être OH ;
$R'_4$ désigne méthyle, phényle ; au moins 60 % en proportion molaire de l'ensemble des radicaux $R_4$ et $R'_4$ désignant méthyle ;
$R_5$ désigne alkyle ou alcényle en $C_8$-$C_{20}$ ;
R" désigne un radical alkylène hydrocarboné divalent, linéaire ou ramifié en $C_2$-$C_{18}$ ;
r est compris entre 1 et 120 inclus ;
p est compris entre 1 et 30 ;
q est égal à 0 ou est inférieur à 0,5 p, p + q étant compris entre 1 et 30 ; les polyorganosiloxanes de formule (XII) peuvent contenir des groupements :

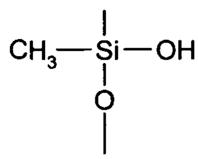

dans des proportions ne dépassant pas 15 % de la somme p + q + r.

- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :

a) 50 à 90% en poids d'acrylate de tertiobutyle ;
b) 0 à 40% en poids d'acide acrylique ;
c) 5 à 40% en poids de macromère siliconé de formule :

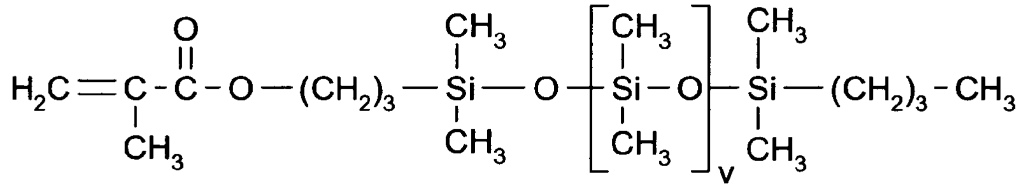

(XIII)

avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions, de nanoémulsions ou de micrémulsions.

Les polyorganosiloxanes particulièrement préférés conformément à l'invention sont :

- les silicones non volatiles choisies dans la famille des polyalkylsiloxanes à groupements terminaux triméthylsilyle telles que les huiles ayant une viscosité comprise entre 0,2 et 2,5 $m^2/s$ à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHODIA CHIMIE, les polyalkylsiloxanes à groupements terminaux diméthylsilanol tels que les diméthiconol ou les polyalkylarylsiloxanes tels que l'huile SILBIONE 70641 V 200 commercialisée par la société RHODIA CHIMIE ;
- la résine d'organopolysiloxane commercialisée sous la dénomination DOW CORNING 593 ;
- les polysiloxanes à groupements aminés tels que les amodiméthicones ou les triméthylsilylamodiméthicone ;

Les protéines ou hydrolysats de protéines cationiques sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :

- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la Société MAYBROOOK et dénommés dans le dictionnaire CTFA "Steartrimonium Hydrolyzed Collagen" ;
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :

- le "Croquat L" dont les groupements ammonium quaternaires comportent un groupement alkyle en $C_{12}$ ;
- le "Croquat M" dont les groupements ammonium quaternairees comportent des groupements alkyle en $C_{10}$-$C_{18}$ ;
- le "Croquat S" dont les groupements ammonium quaternaires comportent un groupement alkyle en $C_{18}$ ;
- le "Crotein Q" dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule (XIV) :

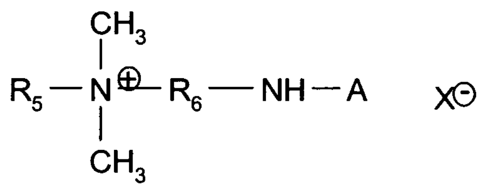

(XIV)

dans laquelle X- est un anion d'un acide organique ou minéral, A désigne un reste de protéine dérivé d'hydrolysats de protéine de collagène, $R_5$ désigne un groupement lipophile comportant jusqu'à 30 atomes de carbone, $R_6$ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quaternisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Selon la présente invention, Les composés de type céramide sont notamment les céramides et/ou les glycocéramides et/ou les pseudocéramides et/ou les néocéramides, naturelles ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

Des composés de type céramides particulièrement préférés selon l'invention sont par exemple :

- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine

ou les mélanges de ces composés.

On peut également utiliser des tensioactifs cationiques parmi lesquels on peut citer en particulier : les sels d'amines grasses primaires, secondaires ou tertiaires, éventuellement polyoxyalkylénées ; les sels d'ammonium quaternaire ; les dérivés d'imidazoline ; ou les oxydes d'amines à caractère cationique.

Les sels d'ammonium quaternaires sont par exemple :

- ceux qui présentent la formule générale (XV) suivante :

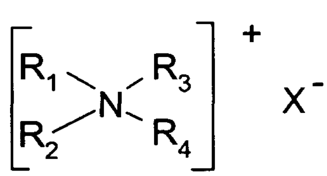

(XV)

dans laquelle les radicaux $R_1$ à $R_4$, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène($C_2$-$C_6$), alkylamide, alkyl($C_{12}$-$C_{22}$)amido alkyle($C_2$-$C_6$), alkyl($C_{12}$-$C_{22}$)acétate, hydroxyalkyle, comportant environ de 1 à 30 atomes de carbone; X est un anion choisi dans le groupe des halogénures, phosphates, acétates,

lactates, alkyl($C_2$-$C_6$)sulfates, alkyl-ou-alkylarylsulfonates,

- les sels d'ammonium quaternaire de l'imidazolinium, comme par exemple celui de formule (XVI) suivante :

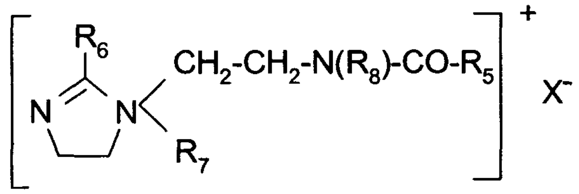

(XVI)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en $C_1$-$C_4$ , $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple commercialisé sous la dénomination «REWOQUAT W 75» par la société DEGUSSA,

- les sels de diammonium quaternaire de formule (XVII) :

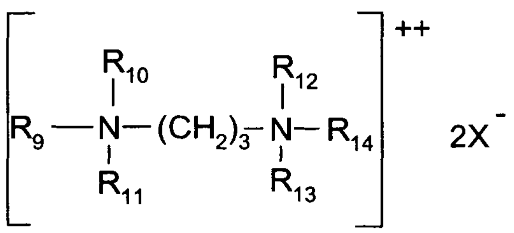

(XVII)

dans laquelle $R_9$ désigne un radical aliphatique comportant environ de 16 à 30 atomes de carbone, $R_{10}$, $R_{11}$, $R_{12}$, $R_{13}$ et $R_{14}$ , identiques ou différents sont choisis parmi l'hydrogène
ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

- les sels d'ammonium quaternaire contenant au moins une fonction ester

Les sels d'ammonium quaternaire contenant au moins une fonction ester utilisables selon l'invention sont par exemple ceux de formule (XVIII ) suivante :

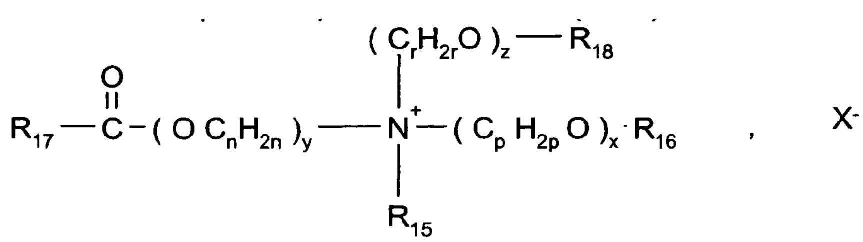

(XVIII)

dans laquelle :

- $R_{15}$ est choisi parmi les radicaux alkyles en $C_1$-$C_6$ et les radicaux hydroxyalkyles ou dihydroxyalkyles en $C_1$-$C_6$ ;
- $R_{16}$ est choisi parmi :

- le radical

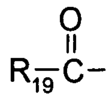

- les radicaux $R_{20}$ hydrocarbonés en $C_1$-$C_{22}$ linéaires ou ramifiés, saturés ou insaturés,
- l'atome d'hydrogène,

- $R_{18}$ est choisi parmi :

  - le radical

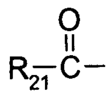

  - les radicaux $R_{22}$ hydrocarbonés en $C_1$-$C_6$ linéaires ou ramifiés, saturés ou insaturés,
  - l'atome d'hydrogène,

- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;

sous réserve que la somme x + y + z vaut de 1 à 15 , que lorsque x vaut 0 alors $R_{16}$ désigne $R_{20}$ et que lorsque z vaut 0 alors $R_{18}$ désigne $R_{22}$.

Les radicaux alkyles $R_{15}$ peuvent être linéaires ou ramifiés et plus particulièrement linéaires.

De préférence $R_{15}$ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque $R_{16}$ est un radical $R_{20}$ hydrocarboné, il peut être long et avoir de 12 à 22 atomes de carbone ou court et avoir de 1 à 3 atomes de carbone.

Lorsque $R_{18}$ est un radical $R_{22}$ hydrocarboné, il a de préférence 1 à 3 atomes de carbone.

Avantageusement, $R_{17}$, $R_{19}$ et $R_{21}$, identiques ou différents, sont choisis parmi les radicaux hydrocarbonés en $C_{11}$-$C_{21}$, linéaires ou ramifiés, saturés ou insaturés, et plus particulièrement parmi les radicaux alkyle et alcényle en $C_{11}$-$C_{21}$, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement les sels d'ammonium de formule (XVIII) dans laquelle :

- $R_{15}$ désigne un radical méthyle ou éthyle,
- x et y sont égaux à 1 ;
- z est égal à 0 ou 1 ;
- n, p et r sont égaux à 2
- n, p et r sont égaux à 2 ;
- $R_{16}$ est choisi parmi :

  - le radical

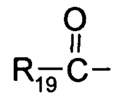

- les radicaux méthyle, éthyle ou hydrocarbonés en $C_{14}$-$C_{22}$
- l'atome d'hydrogène ;

- $R_{18}$ est choisi parmi :

- le radical

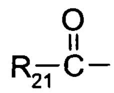

- l'atome d'hydrogène ;

$R_{17}$, $R_{19}$ et $R_{21}$, identiques ou différents, sont choisis parmi les radicaux hydrocarbonés en $C_{13}$-$C_{17}$, linéaires ou ramifiés, saturés ou insaturés et de préférence parmi les radicaux alkyles et alcényle en $C_{13}$-$C_{17}$, linéaires ou ramifiés, saturés ou insaturés.
Avantageusement, les radicaux hydrocarbonés sont linéaires.

On peut citer par exemple les composés de formule (XVI) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société COGNIS, STEPANQUAT par la société STEPAN, NOXAMIUM par la société CECA, REWOQUAT WE 18 par la société DEGUSSA.

On peut aussi utiliser les sels d'ammonium contenant au moins une fonction ester décrits dans les brevets US-A-4874554 et US-A-4137180.

Parmi les sels d'ammonium quaternaire de formule (XV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Les acides gras sont choisis plus particulièrement parmi l'acide myristique, l'acide palmitique, l'acide stéarique, l'acide béhénique, l'acide oléïque, l'acide linoléique, l'acide linolénique et l'acide isostéarique,

Les dérivés d'acides gras sont notamment les esters d'acides carboxyliques en particulier les esters mono, di, tri ou tétracarboxyliques.
Les esters d'acides monocarboxyliques sont notamment les monoesters d'acides aliphatiques saturés ou insaturés, linéaires ou ramifiés en $C_1$-$C_{26}$ et d'alcools aliphatiques saturés ou insaturés, linéaires ou ramifiés en $C_1$-$C_{26}$ , le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle

acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle, le néopentanoate d'isostéaryle, le néopentanoate d'isodécyle.

On peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle, le néopentanoate d'isostéaryle, le néopentanoate d'isodécyle.

Les huiles fluorées sont par exemple les perfluoropolyéthers décrits notamment dans la demande de brevet EP-A-486135 et les composés fluorohydrocarbonées décrites notamment dans la demande de brevet WO 93/11103. L'enseignement de ces deux demandes est totalement inclus dans la présente demande à titre de référence.

Le terme de composés fluorohydrocarbonés désigne des composés dont la structure chimique comporte un squelette carboné dont certains atomes d'hydrogène ont été substitués par des atomes de fluor.

Les huiles fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers ;

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Il est bien entendu possible de mettre en oeuvre des mélanges d'agents conditionneur.

Selon l'invention, le ou les agents conditionneurs peuvent représenter de 0,001 % à 20 % en poids, de préférence de 0,01 % à 10% en poids et plus particulièrement de 0,1 à 3% en poids par rapport au poids total de la composition finale.

Les compositions de l'invention contiennent en outre avantageusement au moins un agent tensioactif qui est généralement présent en une quantité comprise entre 0,1% et 60% en poids environ, de préférence entre 1 % et 40% et encore plus préférentiellement entre 5% et 30%, par rapport au poids total de la composition.

Cet agent tensioactif peut être choisi parmi les agents tensioactifs anioniques, amphotères, non-ioniques, ou leurs mélanges.

Les tensioactifs convenant à la mise en oeuvre de la présente invention sont notamment les suivants :

(i) <u>Tensioactif(s) anionique(s)</u> :

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_6$-$C_{24}$) éther carboxyliques polyoxyalkylénés,

les acides alkyl($C_6$-$C_{24}$)aryl éther carboxyliques polyoxyalkylénés ,les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

(ii) Tensioactif(s) non ionique(s):

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensioactifs non-ioniques rentrant particulièrement bien dans le cadre de la présente invention.

(iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits commercialisés sous les dénomination MIRANOL, tels que décrits dans les brevets US-2 528 378 et US-2 781 354 et de structures :

$$R_2\text{ -CONHCH}_2\text{CH}_2\text{ -N(R}_3\text{)(R}_4\text{)(CH}_2\text{COO-)} \qquad (2)$$

dans laquelle : $R_2$ désigne un radical alkyle dérivé d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ;
et

$$R_5\text{-CONHCH}_2\text{CH}_2\text{-N(B)(C)} \qquad (3)$$

dans laquelle :

B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$ - CHOH - SO3H
$R_5$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL C2M concentré par la société RHODIA CHIMIE.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl($C_{12}$-$C_{14}$) sulfates de sodium, de triéthanolamine ou d'ammonium, les alkyl ($C_{12}$-$C_{14}$)éthersulfates de sodium, de triéthanolamine ou d'ammonium oxyéthylénés à 2,2 moles d'oxyde d'éthylène, le cocoyl iséthionate de sodium et l'alphaoléfine($C_{14}$-$C_{16}$) sulfonate de sodium et leurs mélange avec :

- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société COGNIS.

La composition de l'invention peut également contenir au moins un additif choisi parmi les épaississants, les parfums, les agents nacrants, les conservateurs, les polymères anioniques ou non ioniques, les protéines non cationiques, les hydrolysats de protéines non cationiques, l'acide méthyl-18 eicosanoique, les hydroxyacides, d'autres polymères que ceux de l'invention et en particulier des polyuréthanes associatifs cationiques ou non ioniques polyéther et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères, non ioniques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale. De préférence, la base lavante contient au moins 3% en poids de tensioactif anioniques, plus particulièrement de 4 à 30% en poids par rapport au poids total de la composition.

Le pH de la composition appliquée sur les matières kératiniques est généralement compris entre les valeurs 2 et 11. Il est de préférence compris entre 3 et 8, et peut être ajusté à la valeur désirée au moyen d'agents acidifiants ou alcalinisants bien connus de l'état de la technique des compositions appliquées sur des matières kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XXI suivante :

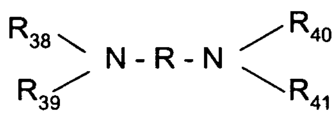

(XX)

dans laquelle R est un reste propylène éventuellement substitué par un groupement hydroxyle ou un radical alkyle en $C_1$-$C_4$ ; $R_{38}$, $R_{39}$, $R_{40}$ et $R_{41}$, identiques ou différents, représentent un atome d'hydrogène, un radical alkyle en $C_1$-$C_4$

ou hydroxyalkyle en $C_1$-$C_4$.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le milieu physiologiquement et en particulier cosmétiquement acceptable peut être constitué uniquement par de l'eau, par un solvant cosmétiquement acceptable ou par un mélange d'eau et d'un solvant cosmétiquement acceptable tel que notamment un alcool inférieur en $C_1$-$C_4$, comme l'éthanol, l'isopropanol, le tertiobutanol, le n-butanol ; les polyols comme le propylèneglycol, la glycérine, le diéthylèneglycol, les éthers de polyols.

L'invention a aussi pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits. Dans les exemples, MA signifie matière active.

EXEMPLE 1

On a réalisé une composition de shampooing :

| | En g |
|---|---|
| | |
| Chlorure de Guar Hydroxypropyltrimonium | 0,05 |
| Coco-Betaine ( 32 % MA ) | 9 |
| Lauryl éther (2OE) sulfate de sodium (70 % MA) | 22,2 |
| Sodium Methyl Paraben | 0,2 |
| DMDM Hydantoine | 0,25 |
| acide mucique (Acide Galactarique) | 0,3 |
| Dimethicone ( DC 200 Fluid 300000 de Dow Corning) | 2,7 |
| Mélange d'alcool cétylique et de 1-(hexadécyloxy)-2-octadécanol | 2,5 |
| Parfum | 0,5 |
| Monoisopropanolamide de coprah (cocamide MIPA) | 0,3 |
| Carbomer | 0,2 |
| Hydroxyde de sodium q.s | pH 7 |
| Eau qsp | 100 |

Les cheveux traités avec ce shampooing sont souples et disciplinés.

EXEMPLE 2

: Compositions oxydantes à base d'eau oxygénée prêtes à l'emploi pour un usage en coloration, en décoloration et en déformation permanente des fibres kératiniques.

On a préparé deux compositions oxydantes prêtes à l'emploi - respectivement B et C - pour la coloration, la décoloration et la déformation permanente de fibres kératiniques. Leur composition qualitative et quantitative est présentée dans le tableau 1 ci-après, dans lequel les quantités des différents constituants sont exprimées en grammes.

TABLEAU I

| Constituants | Composition B | Composition C |
|---|---|---|
| Alcool cétylique | 3 | 3 |
| Lauryl sulfate de sodium | 0,5 | 0,5 |
| Alcool oléique polyglycolé (2 moles) | 0,45 | 0,45 |
| Alcool oléique polyglycérolé (4 moles) | 0,35 | 0,35 |
| Siméthicone | 0,045 | 0,045 |
| Acide mucique* | 0,02 | 0,03 |
| Pyrophosphate tétrasodique, $10H_2O$ | 0,02 | 0,04 |
| Salicylate de sodium | - | 0,035 |
| Stannate de sodium | 0,04 | - |
| Eau oxygénée à 50% | 24 | 18 |
| Acide phosphorique à 85% en solution aqueuse | q.s.p pH=2 | q.s.p pH=2 |
| Eau | q.s.p 100 g | q.s.p 100 g |

* Muciliance - Société SOLIANCE

EXEMPLE 3

On a préparé, dans cet exemple, deux compositions -respectivement D et E- destinées à un usage pour la décoloration, ces compositions étant toutes deux des compositions anhydres, sous forme pulvérulente, comprenant un agent complexant conforme à l'invention.
Le tableau II représente les compositions qualitatives et quantitatives de ces compositions, les quantités étant exprimées en pourcentage massique.

TABLEAU II

| Constituants | Composition D | Composition E |
|---|---|---|
| Persulfate de potassium | 39,5 | 46 |
| Persulfate de sodium | 30 | 15 |
| Disilicate de sodium | - | 15 |
| Métasilicate de sodium | 14 | 4 |
| Chlorure d'ammonium | 6 | 4 |
| Urée | - | 4,5 |
| Acide mucique* | 1 | 0,8 |
| Copolymère hexaméthyl diisocyanate/polyéthylèneglycol à terminaison $\alpha$ et $\omega$ stéaryl polyoxyéthylène** | 0,5 | - |

*Muciliance - Société SOLIANCE
**SER-AD FX 1100-Société SERVO DELDEN ;

TABLEAU II (suite)

| Constituants | Composition D | Composition E |
|---|---|---|
| Copolymère acide acrylique/méthacrylate d'alkyl (C10/C30) réticulé*** | - | 1 |
| Carboxyméthyl amidon de pommme de terre/sel de sodium faiblement réticulé | - | 2 |
| Gomme de Guar | 2 | 1,5 |
| Colorant (ultramarine) | 0,5 | - |
| Oxyde de titane | 0,5 | 1 |
| Lauryl sulfate de sodium | 2 | 2 |
| Stéarate de calcium | 1 | 1 |
| Silice pyrogénée à caractère hydrophile | 3 | 0,2 |
| Polydécène hydrogéné**** | - | 2 |

***CARBOPOL ETD 2020-Société NOVEON ;
****SILKFLO 366 NF POLYDECECE-Société AMOCO CHEMICAL

La composition de décoloration D (40g) est mélangée avec la composition oxydante à base d'eau oxygénée A (80 g). On applique le mélange décolorant prêt à l'emploi ainsi obtenu 45 minutes sous casque sur cheveux naturels foncés, puis on rince abondamment à l'eau. A l'issue de ces opérations, on obtient une décoloration puissante et homogène.

La composition de décoloration E (40g) est mélangée avec la composition oxydante à base d'eau oxygénée C (60g). On applique le mélange décolorant prêt à l'emploi ainsi obtenu 30 minutes sous casque sur cheveux naturels foncés, puis on rince abondamment à l'eau. A l'issue de ces opérations, on obtient une décoloration puissante et homogène, avec des cheveux doux et brillants et faciles à démêler.

EXEMPLE 4.

On a préparé, dans cet exemple, deux compositions -respectivement F et G- destinées à un usage pour la décoloration, ces compositions étant toutes deux des compositions anhydres, sous forme de pâte, comprenant un agent complexant conforme à l'invention.

Le tableau III représente les compositions qualitatives et quantitatives de ces compositions, les quantités étant exprimées en pourcentage massique.

TABLEAU III

| Constituants | Composition F | Composition G |
|---|---|---|
| Persulfate de potassium | 35,8 | 35,6 |
| Persulfate de sodium | 6 | 6 |
| Disilicate de sodium | 15 | 15 |
| Métasilicate de sodium | 3 | 3 |
| Chlorure d'ammonium | 4,2 | 4,2 |
| Acide mucique* | 1 | 1 |
| Copolymère hexaméthyl diisocyanate/polyéthylèneglycol à terminaison $\alpha$ et $\omega$ stéaryl polyoxyéthylène** | 2 | 0,5 |
| Copolymère acide acrylique/méthacrylate d'alkyl (C10/C30) réticulé*** | - | 0,5 |
| Carboxyméthyl amidon de pommme de terre/sel de sodium faiblement réticulé | 2 | 1 |
| Gomme de Guar | - | 2 |

*Muciliance - Société SOLIANCE
**SER-AD FX 1100-Société SERVO DELDEN ;
***CARBOPOL ETD 2020-Société NOVEON ;

TABLEAU III (suite)

| Constituants | Composition F | Composition G |
|---|---|---|
| Colorant (ultramarine) | 0,5 | 0,5 |
| Oxyde de titane | 1 | 1 |
| Lauryl sulfate de sodium | 3,5 | 3,5 |
| Stéarate de calcium | 2 | 2 |
| Silice pyrogénée à caractère hydrophile | 0,5 | 0,5 |
| Palmitate d'isopropyle | 22,5 | - |
| Cire d'abeille | 1 | - |
| Polydécène hydrogéné**** | - | 23 |
| Silice pyrogénée à caractère hydrophobe | - | 0,7 |

****SILKFLO 366 NF POLYDECECE-Société AMOCO CHEMICAL

La composition de décoloration F (40g) est mélangée avec la composition oxydante à base d'eau oxygénée A (80 g). On applique le mélange décolorant prêt à l'emploi ainsi obtenu 45 minutes sous casque sur cheveux naturels foncés, puis on rince abondamment à l'eau. A l'issue de ces opérations, on obtient une décoloration puissante et homogène avec des cheveux doux et brillants et faciles à démêler.

La composition de décoloration G (40g) est mélangée avec la composition oxydante à base d'eau oxygénée C (60g). On applique le mélange décolorant prêt à l'emploi ainsi obtenu 25 minutes sous casque sur cheveux naturels foncés, puis on rince abondamment à l'eau. A l'issue de ces opérations, on obtient une décoloration puissante et homogène, avec des cheveux doux et brillants et faciles à démêler.

EXEMPLE 5.

Dans cet exemple, dans un premier temps, on prépare une composition réductrice.

Le tableau IV représente les compositions qualitatives et quantitatives de ces compositions, les quantités étant exprimées en pourcentage massique.

TABLEAU IV

| Constituants | Composition H |
|---|---|
| Acide thioglycolique | 9,2 |
| Arginine | 15 |
| Ammoniaque à 20% | 1,86 |
| Carbonate d'ammonium | 4,5 |
| Cocoylamidopropylbétaïne/momolaurate de glycérol (25/5) en solution aqueuse à 30% | 1,3 |
| Peptisant | 0,8 |
| Alcool isostéarylique | 12 |
| Agent complexant | 0,4 |
| Parfum | 0,4 |
| Eau | q.s.p 100g |

La composition H est appliquée sur une mèche de cheveux humides, préalablement enroulée sur un bigoudi de 9 mm de diamètre, le temps de pose étant de 10 minutes. Puis, on rince les mèches ainsi traitées abondamment à l'eau.

Dans un deuxième temps, on prépare une composition oxydante, dite composition 1.

Le tableau V représente les compositions qualitatives et quantitatives de ces compositions, les quantités étant exprimées en pourcentage massique.

TABLEAU V

| Constituants | Composition 1 |
|---|---|
| Alcool cétylique | 3 |
| Lauryl sulfate de sodium | 0,5 |
| Alcool oléique polyglycérolé (2 moles) | 0,45 |
| Alcool oléique polyglycérolé (4 moles) | 0,35 |
| Siméthicone | 0,045 |
| Acide mucique* | 0,03 |
| Pyrophosphate tétrasodique, $10H_2O$ | 0,02 |
| Salicylate de sodium | - |
| Stannate de sodium, $6H_2O$ | 0,04 |
| Eau oxygénée à 50% | 5,4 |
| Acide phosphorique à 85% en solution aqueuse | q.s.p pH=2 |
| Eau | q.s.p 100g |

* Musiliance - Société SOLIANCE

La composition I est appliquée sur les mèches précédemment traités par la composition réductrice H, par application de ladite composition S pendant un temps de pose de 10 minutes. Ensuite, les mèches sont rincées abondamment à l'eau. Enfin, les cheveux sont déroulés du bigoudi, puis séchés. La mèche ainsi traitée est ondulée et douce.

EXEMPLE 6.

Dans cet exemple, dans un premier temps, on prépare une composition tinctoriale J.
Le tableau VI représente les compositions qualitatives et quantitatives de cette composition, les quantités étant exprimées en pourcentage massique.

TABLEAU VI

| Constituants | Composition J |
|---|---|
| Paraphénylènediamine | 0,108 |
| 2-méthyl-5-amino-phénol | 0,123 |
| Ethanol | 20 |
| Alcool benzylique | 2 |
| Alkyl (C8/C10) polyglucoside en solution aqueuse à 60% | 3,6 |
| Polyéthylèneglycol à 8 moles d'oxyde d'éthylène | 3 |
| Métabisulfite de sodium en solution aqueuse à 35% | 3 |
| Complexant | 0,227 |
| Ammoniaque à 20% | 0,48 |
| Eau | 6,8 |

On prépare dans un deuxième temps la composition oxydante K.
La tableau VII représente les compositions qualitatives et quantitatives de cette composition, les quantités étant exprimées en pourcentage massique.

TABLEAU VII

| Constituants | Composition A |
|---|---|
| Alcool cétylique | 3 |
| Lauryl sulfate de sodium | 0,5 |
| Alcool oléique polyglycolé (2 moles) | 0,45 |
| Alcool oléique polyglycérolé (4 moles) | 0,35 |
| Siméthicone | 0,045 |
| Acide mucique* | 0,1 |
| Pyrophosphate tétrasodique, $10H_2O$ | 0,02 |
| Salicylate de sodium | - |
| Stannate de sodium | 0,04 |
| Eau oxygénée à 50% | 24 |
| Acide phosphorique à 85% en solution aqueuse | q.s.p pH=2 |
| Eau | q.s.p 100 g |

* Muciliance - Société SOLIANCE
** Gluconal® GA-50-SG - Société AKZO NOBEL

La composition tinctoriale J est mélangée avec la composition oxydante à base d'eau oxygénée K, en poids égale.

La composition résultante est appliquée pendant 30 minutes sur des mèches de cheveux gris naturels à 90% de blancs, puis les cheveux ainsi traitées sont rincés abondamment, lavés avec un shampooing standard, rincés à nouveau puis séchés.

EXEMPLE 7

On prépare la formulation suivante, se trouvant sous forme de crème :

| | |
|---|---|
| Alcool cétylstéarylique | 13 % |
| Alcool laurique oxyéthyléné (12OE) | 8 % |
| Alcool décylique oxyéthyléné (3OE) à 90 % dans l'eau [Empilan KA 2,5/90FL - commercialisé par HUNSTMANN] | 6 % |
| Alcool oléocétylique oxyéthyléné (30OE) | 4 % |
| Acide laurique | 5 % |
| Monoéthanolamine | 2 % |
| Propylène glycol | 8 % |
| Polycondensat tétraméthyl héxaméthylène diamine / dichloro 1,3-propylène à 60 % dans l'eau [Mexomere PO - commercialisé par CHIMEX] | 1 % |
| Copolymère chlorure de diméthyl diallylammonium / acide acrylique (80/20) à 40,5 % dans l'eau [Merquat 280 - commercialisé par Nalco] | 2 % |
| Distéarate de glycol | 4 % |
| Silice pyrogénée à caractère hydrophobe | 2 % |
| Acide polyacrylique réticulé [Carbopol 980 - commercialisé par Noveon] | 0,6 % |
| Acide mucique | 1 % |
| 1,3-dihydroxybénezène (résorcinol) | 0,67 % |

(suite)

| | |
|---|---|
| Paraphénylènediamine | 0,88 % |
| 5-N-(β-hydroxyéthyl)amino-2-méthyl-phénol | 0,055 % |
| 2-méthyl-1,3-dihydroxy benzène | 0,11 % |
| Para-aminophénol | 0,27 % |
| 4-(méthylamino)phénol hémisulfate | 0,26 % |
| 1-hdyroxy-3-amino benzène | 0,16 % |
| Antioxydant | qs |
| Réducteur | qs |
| Parfum | qs |
| Ammoniaque (20% NH3) | 11,1 % |
| Eau | qsp 100% |

Les pourcentages sont exprimés en poids.
Au moment de l'emploi, cette composition est mélangée avec une fois et demi son poids d'une composition oxydante titrant 6 % en peroxyde d'hydrogène et le mélange obtenu est appliqué sur cheveux pendant 30 minutes puis rincé.
On obtient une nuance peu sélective.

## Revendications

1. Composition de traitement des matières kératiniques, en particulier des fibres kératiniques humaines telles que les cheveux, comprenant dans un milieu physiologiquement et en particulier cosmétiquement acceptable au moins un acide hydroxycarboxylique ou ses sels répondant à la formule générale (1) suivante :

$$R\text{-}(CHOH)_4\text{-}CO_2X \qquad (I)$$

dans laquelle :

- R représente un groupe $CO_2X$, et
- X représente un atome d'hydrogène ou un cation monovalent ou divalent issu d'un métal de transition, d'un métal alcalin ou alcalinoterreux, d'une amine organique ou d'un ion ammonium,

et au moins un agent protecteur et/ou un agent conditionneur, ledit agent conditionneur étant choisi parmi les huiles de synthèse, les huiles végétales, les huiles fluorées ou perfluorées, les cires naturelles ou synthétiques, les silicones, les polymères cationiques non polysaccharides, les composés de type céramide, les tensioactifs cationiques, les amines grasses, les acides gras et leurs dérivés, ainsi que les mélanges de ces différents composés.

2. Composition selon la revendication 1, dans laquelle le cation monovalent ou divalent est choisi dans le groupe constitué par les cations de métaux alcalins, les cations de métaux alcalino-terreux et les cations divalents de métaux de transition.

3. Composition selon la revendication 1 ou la revendication 2, dans laquelle le ou les composés de formule (1) sont choisis dans le groupe constitué par l'acide mucique, l'acide glucarique, l'acide mannarique, leurs sels de métaux alcalins, leurs sels de métaux alcalino-terreux, leurs sels de métaux de transition, et leurs mélanges.

4. Composition selon l'une quelconque des revendications précédentes, dans laquelle le composé de formule (1) est l'acide mucique.

**5.** Composition selon l'une quelconque des revendications précédentes, dans laquelle le ou les composés de formule (1) représentent de 0,001 à 10% en poids du poids total de la composition.

**6.** Composition selon l'une quelconque des revendications précédentes, dans laquelle le ou les composés de formule (1) représentent de 0,001 à 5% en poids du poids total de la composition.

**7.** Composition selon l'une quelconque des revendications précédentes, **caractérisée par le fait que** ledit agent protecteur des matières kératiniques est choisi parmi les filtres UV, les agents antiradicalaires, les agents antioxydants, les vitamines et les provitamines.

**8.** Composition selon la revendication précédente, **caractérisée par le fait que** lesdits filtres UV sont choisis parmi les filtres organiques hydrosolubles, liposolubles ou insolubles dans l'eau, siliconés ou non siliconés et les nanoparticules d'oxydes minéraux dont la surface a éventuellement été traitée.

**9.** Composition selon la revendication précédente, **caractérisée par le fait que** ledit filtre UV organiques hydrosoluble est choisi parmi dans le groupe constitué par l'acide para-aminobenzoïque et ses sels, l'acide anthranilique et ses sels, l'acide salicylique et ses sels, l'acide p-hydroxycinnamique et ses sels, les dérivés sulfoniques de benzoxazole et leurs sels, les dérivés sulfoniques du benzophénone et leurs sels, les dérivés sulfoniques de benzylidène camphre et leurs sels, les dérivés de benzylidène camphre substitués par une amine quaternaire et leurs sels, les dérivés des acides phtalydène-camphosulfoniques et leurs sels, les dérivés sulfoniques de benzotriazole, les polymères hydrophiles présentant, en outre et de par leur nature chimique, des propriétés de photoprotection contre le rayonnement UV leurs mélanges.

**10.** Composition selon la revendication 8, **caractérisée en ce que** ledit filtre UV organique liposoluble est choisi parmi : les dérivés d'acide p-aminobenzoïque tels que les esters ou amides d'acide p-aminobenzoïque ; les dérivés d'acide salicylique tels que les esters d'acide salicylique ; les dérivés de benzophénone ; les dérivés de dibenzoylméthane ; les dérivés de diphénylacrylates ; les dérivés de benzofurannes ; les filtres UV polymères contenant un ou plusieurs résidus silico-organiques ; les esters d'acide cinnamique ; les dérivés de camphre ; les dérivés de trianilino-s-triazine ; l'ester éthylique de l'acide urocanique ; les benzotriazoles ; les dérivés d'hydroxyphényltriazine ; les bis-résorcinol-dialkylaminotriazine ; et leurs mélanges.

**11.** Composition selon la revendication 10, **caractérisée en ce que** ledit filtre UV liposoluble est choisi parmi : l'octyl salicylate ; le 2-hydroxy-4-méthoxybenzophénone; le 4-tertiobutyl 4'-méthoxydibenzoylméthane ; l'octocrylène ; le 4-méthoxy cinnamate de 2-éthylhexyl; et le composé de formule (II) :

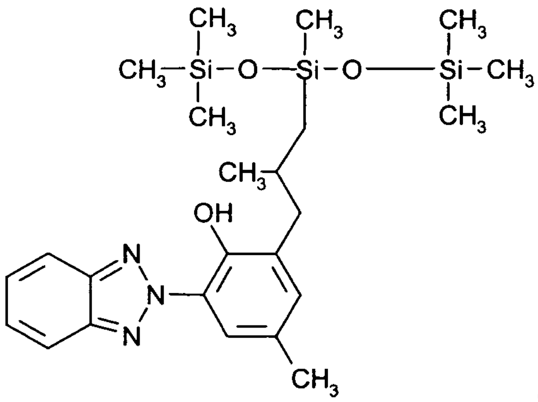

(II)

**12.** Composition selon l'une quelconques des revendications 1 à 11, **caractérisée par le fait que** les huiles de synthèse sont des polyoléfines de type polybutène, hydrogéné ou non, ou de type polydécène, hydrogéné ou non.

**13.** Composition selon l'une quelconques des revendications 1 à 12, **caractérisée par le fait que** les polymères cationiques sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

**14.** Composition selon l'une quelconque des revendications 1 ou 13, **caractérisée par le fait que** ledit polymère cationique est choisi parmi les cyclopolymères cationiques, les polymères quaternaires de vinylpyrrolidone et de vinylimidazole et leurs mélanges.

**15.** Composition selon la revendication 14, **caractérisée par le fait que** ledit cyclopolymère est choisi parmi les homopolymères du chlorure de diallyldiméthylammonium et les copolymères du chlorure de diallyldiméthylammonium et d'acrylamide.

**16.** Composition selon l'une quelconque des revendications 1 à 15, **caractérisée par le fait que** les silicones sont choisies parmi les polyorganosiloxanes insolubles dans la composition.

**17.** Composition selon l'une quelconque des revendications 1 ou 16, **caractérisée par le fait que** les silicones sont des polyorganosiloxanes non volatils choisis parmi les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

**18.** Composition selon la revendication 17, **caractérisée par le fait que** :

(a) les polyalkylsiloxanes sont choisis parmi :

- les polydiméthylsiloxanes à groupements terminaux triméthylsilyle ;
- les polydiméthylsiloxanes à groupements terminaux diméthylsilanol ;
- les polyalkyl($C_1$-$C_{20}$)siloxanes ;

(b) les polyalkylarylsiloxanes sont choisis parmi :

- les polydiméthylméthylphénylsiloxanes, les polydiméthyldiphényl siloxanes linéaires et/ou ramifiés de viscosité comprise entre $1.10^{-5}$ et $5.10^{-2} m^2/s$ à 25°C ;

(c) les gommes de silicone sont choisies parmi les polydiorganosiloxanes ayant des masses moléculaires moyennes en nombre comprises entre 200 000 et 1 000 000 utilisés seuls ou sous forme de mélange dans un solvant ;
(d) les résines sont choisies parmi les résines constituées d'unités : $R_3$ Si $O_{1/2}$, $R_2$ Si $O_{2/2}$, R Si $O_{3/2}$, Si $O_{4/2}$ dans lesquelles R représente un groupement hydrocarboné ayant de 1 à 16 atomes de carbone ou un groupement phényle ;
(e) les silicones organo modifiées sont choisies parmi les silicones comportant dans leur structure un ou plusieurs groupements organofonctionnels fixés par l'intermédiaire d'un radical hydrocarboné.

**19.** Composition selon la revendication 18, **caractérisée par le fait que** les gommes de silicone utilisées seules ou sous forme de mélange sont choisies parmi les structures suivantes :

- polydiméthylsiloxane
- polydiméthylsiloxane/méthylvinylsiloxanes,
- polydiméthylsiloxane/diphénylsiloxane,
- polydiméthylsiloxane/phénylméthylsiloxane,
- polydiméthylsiloxane/diphénylsiloxane/méthylvinylsiloxanes et les mélanges suivants :

- des mélanges formés à partir d'un polydiméthylsiloxane hydroxylé en bout de chaîne et d'un polydiméthylsiloxane cyclique ;
- les mélanges formés à partir d'une gomme polydiméthylsiloxane et d'une silicone cyclique ; et
- des mélanges de polydiméthylsiloxanes de viscosités différentes.

**20.** Composition selon la revendication 18, **caractérisée par le fait que** les silicones organomodifiées sont choisies parmi les polyorganosiloxanes comportant :

a) des groupements polyéthylèneoxy et/ou polypropylèneoxy ;
b) des groupements aminés substitués ou non ;
c) des groupements thiols ;

d) des groupements alcoxylés,
e) des groupements hydroxyalkyle,
f) des groupements acyloxyalkyle,
g) des groupements alkyl carboxyliques,
h) des groupements 2-hydroxyalkylsulfonates,
i) des groupements 2-hydroxyalkylthiosulfonates,
j) des groupements hydroxyacylamino.

**21.** Composition selon l'une quelconque des revendications 1 à 20, **caractérisée par le fait que** les polyorganosiloxanes sont choisis parmi les polyalkylsiloxanes à groupements terminaux triméthylsilyle, les polyalkylsiloxanes à groupements terminaux diméthylsilanol, les polyalkylarylsiloxanes, les mélanges de deux PDMS constitués d'une gomme et d'une huile de viscosités différentes, les mélanges d'organosiloxanes et de silicones cycliques, les résines d'organopolysiloxanes.

**22.** Composition selon l'une quelconque des revendications 1 à 21, **caractérisée par le fait que** les composés de type céramides sont choisis parmi :

- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 trio) et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine

ou les mélanges de ces composés.

**23.** Composition selon l'une quelconque des revendications précédentes, **caractérisée en ce que** le ou les agents protecteurs et/ou conditionneurs sont présents à une concentration comprise entre 0,001 % et 20 % en poids par rapport au poids total de la composition, de préférence entre 0,01 % et 10 % en poids.

**24.** Composition selon l'une quelconque des revendications précédentes, **caractérisée par le fait qu'**elle comprend en outre au moins un agent tensioactif choisi parmi les tensioactifs anioniques, non ioniques, amphotères et leurs mélanges.

**25.** Composition selon la revendication 24, **caractérisée par le fait que** le ou les agents tensioactifs additionnels sont présents à une concentration comprise entre 0,1% et 60% en poids, de préférence entre 1% et 40% en poids, et encore plus préférentiellement entre 5% et 30% en poids, par rapport au poids total de la composition.

**26.** Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer avant et/ou après une coloration ou une décoloration, de composition à rincer à appliquer avant et/ou entre les deux étapes d'une permanente ou d'un défrisage, de composition lavantes pour le corps.

**27.** Utilisation d'une composition telle que définie dans l'une quelconque des revendications précédentes pour le lavage ou pour le soin des matières kératiniques.

**28.** Procédé de traitement des matières kératiniques, telles que les cheveux, **caractérisé en ce qu'**il consiste à appliquer sur lesdites matières une composition cosmétique selon l'une des revendications 1 à 26, puis à effectuer éventuellement un rinçage à l'eau.

**29.** Utilisation d'un acide hydroxycarboxylique ou de ses sels tel que défini à l'une des revendications 1 à 9 dans, ou pour la fabrication d'une composition cosmétique comprenant au moins un agent protecteur et/ou conditionneur

tels que définis à la revendication 1.

**30.** Utilisation d'au moins un acide hydroxycarboxylique ou ses sels tel que défini à l'une des revendications 1 à 9 dans une composition comprenant un agent protecteur des matières kératiniques pour augmenter l'efficacité de cet agent protecteur des matières kératiniques.

**31.** Utilisation d'au moins un acide hydroxycarboxylique ou ses sels tel que défini à l'une des revendications 1 à 9 dans une composition comprenant un agent conditionneur des matières kératiniques pour augmenter l'efficacité de cet agent conditionneur des matières kératiniques.

Office européen des brevets

# RAPPORT DE RECHERCHE EUROPEENNE

Numéro de la demande
EP 04 29 0563

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | EP 0 531 111 A (UNILEVER PLC ;UNILEVER NV (NL)) 10 mars 1993 (1993-03-10)<br>* page 4, ligne 17-25; exemples 14-21,25,27 * | 1-29 | A61K7/075<br>A61K7/06 |
| X | EP 0 375 388 A (UNILEVER PLC ;UNILEVER NV (NL)) 27 juin 1990 (1990-06-27)<br>* page 4, ligne 5-35; exemples 2-9,15-19; tableau 2 * | 1-29 | |
| X | WO 03/015730 A (OREAL ;CANNELL DAVID W (US); NGUYEN NGHI VAN (US)) 27 février 2003 (2003-02-27)<br>* alinéas [0024] - [0026]; exemple 3 * | 1-29 | |
| X | EP 0 770 399 A (YU RUEY J ;SCOTT EUGENE J VAN (US)) 2 mai 1997 (1997-05-02)<br>* exemples 1,18 *<br>* page 2, ligne 40-53 *<br>* page 3, ligne 41,42 *<br>* page 4, ligne 37,38 *<br>* page 5, ligne 34,35 * | 1-31 | **DOMAINES TECHNIQUES RECHERCHES (Int.Cl.7)** |
| X | WO 01/97755 A (HENKEL KGAA ;KLEEN ASTRID (DE)) 27 décembre 2001 (2001-12-27)<br>* pages 1-2 *<br>* page 8, ligne 1,2 *<br>* page 10, ligne 29 - page 11, ligne 9 * | 1-31 | A61K |
| X | EP 0 855 178 A (KAO CORP) 29 juillet 1998 (1998-07-29)<br>* page 2, ligne 24-46 * | 1-31 | |
| A | US 5 804 172 A (AULT FREDERICK K) 8 septembre 1998 (1998-09-08)<br>* colonne 1, ligne 20-25,54-61 *<br>* colonne 2, ligne 17-20,40-44 *<br>* colonne 4, ligne 1-7 * | 1-31 | |
| | -/-- | | |

Le présent rapport a été établi pour toutes les revendications

| Lieu de la recherche | Date d'achèvement de la recherche | Examinateur |
|---|---|---|
| Munich | 25 juin 2004 | Sala-Jung, N |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 04 29 0563

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | FR 2 100 198 A (ATLAS CHEM IND) 17 mars 1972 (1972-03-17) * page 1, ligne 22-27 * * page 4; tableaux 1-3 * ----- | 1-31 | |
| | | | DOMAINES TECHNIQUES RECHERCHES (Int.Cl.7) |

Le présent rapport a été établi pour toutes les revendications

| Lieu de la recherche | Date d'achèvement de la recherche | Examinateur |
|---|---|---|
| Munich | 25 juin 2004 | Sala-Jung, N |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 04 29 0563

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

25-06-2004

| Document brevet cité au rapport de recherche | Date de publication | Membre(s) de la famille de brevet(s) | Date de publication |
|---|---|---|---|
| EP 0531111 A | 10-03-1993 | AU 2212192 A | 11-03-1993 |
| | | BR 9203451 A | 06-04-1993 |
| | | CA 2077153 A1 | 05-03-1993 |
| | | EP 0531111 A1 | 10-03-1993 |
| | | JP 5194151 A | 03-08-1993 |
| | | US 5300284 A | 05-04-1994 |
| | | ZA 9206720 A | 04-03-1994 |
| EP 0375388 A | 27-06-1990 | AT 102011 T | 15-03-1994 |
| | | AU 626364 B2 | 30-07-1992 |
| | | AU 4712289 A | 28-06-1990 |
| | | BR 8906716 A | 06-11-1990 |
| | | CA 2005811 A1 | 22-06-1990 |
| | | DE 68913476 D1 | 07-04-1994 |
| | | DE 68913476 T2 | 01-06-1994 |
| | | EP 0375388 A2 | 27-06-1990 |
| | | ES 2062048 T3 | 16-12-1994 |
| | | IN 170996 A1 | 27-06-1992 |
| | | JP 1714853 C | 27-11-1992 |
| | | JP 2221216 A | 04-09-1990 |
| | | JP 4000963 B | 09-01-1992 |
| | | US 5081151 A | 14-01-1992 |
| | | ZA 8909841 A | 28-08-1991 |
| WO 03015730 A | 27-02-2003 | US 2003079299 A1 | 01-05-2003 |
| | | EP 1418878 A1 | 19-05-2004 |
| | | WO 03015730 A1 | 27-02-2003 |
| EP 0770399 A | 02-05-1997 | EP 0770399 A2 | 02-05-1997 |
| | | AU 654850 B2 | 24-11-1994 |
| | | AU 1394392 A | 28-05-1992 |
| | | AU 618517 B2 | 02-01-1992 |
| | | AU 7998687 A | 23-06-1988 |
| | | CA 1324077 C | 09-11-1993 |
| | | CA 1340120 C | 10-11-1998 |
| | | CA 1339706 C | 10-03-1998 |
| | | DE 3751361 D1 | 27-07-1995 |
| | | DE 3752045 D1 | 07-05-1997 |
| | | DE 3752045 T2 | 13-11-1997 |
| | | EP 0273202 A2 | 06-07-1988 |
| | | EP 0599819 A2 | 01-06-1994 |
| | | ES 2074978 T3 | 01-10-1995 |
| | | ES 2103506 T3 | 16-09-1997 |
| | | JP 2533339 B2 | 11-09-1996 |
| | | JP 63166837 A | 11-07-1988 |
| | | US 5665776 A | 09-09-1997 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 04 29 0563

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

25-06-2004

| Document brevet cité au rapport de recherche | Date de publication | Membre(s) de la famille de brevet(s) | Date de publication |
|---|---|---|---|
| EP 0770399 A | | US 2003017130 A1 | 23-01-2003 |
| | | US 5389677 A | 14-02-1995 |
| | | US 5702688 A | 30-12-1997 |
| | | US 5422370 A | 06-06-1995 |
| | | US 5470880 A | 28-11-1995 |
| | | US 6060512 A | 09-05-2000 |
| | | US 6384079 B1 | 07-05-2002 |
| | | US 5547988 A | 20-08-1996 |
| | | US 5091171 A | 25-02-1992 |
| | | US 5561159 A | 01-10-1996 |
| | | US 5591774 A | 07-01-1997 |
| | | US 5550154 A | 27-08-1996 |
| | | US 5589505 A | 31-12-1996 |
| | | US 5561155 A | 01-10-1996 |
| | | US 5670541 A | 23-09-1997 |
| | | US 5668177 A | 16-09-1997 |
| | | US 5827882 A | 27-10-1998 |
| | | US 5580902 A | 03-12-1996 |
| | | US 5670542 A | 23-09-1997 |
| | | US 5612376 A | 18-03-1997 |
| | | US 5674899 A | 07-10-1997 |
| | | US 5643961 A | 01-07-1997 |
| | | US 5648395 A | 15-07-1997 |
| | | US 5643962 A | 01-07-1997 |
| | | US 5643952 A | 01-07-1997 |
| | | US 5656665 A | 12-08-1997 |
| | | US 5677339 A | 14-10-1997 |
| | | US 5574067 A | 12-11-1996 |
| | | US 5650436 A | 22-07-1997 |
| | | US 5637615 A | 10-06-1997 |
| | | US 5643953 A | 01-07-1997 |
| | | US 5556882 A | 17-09-1996 |
| WO 0197755 A | 27-12-2001 | DE 10030313 A1 | 03-01-2002 |
| | | DE 10120305 A1 | 31-10-2002 |
| | | AU 6395501 A | 02-01-2002 |
| | | AU 6605701 A | 02-01-2002 |
| | | AU 6753301 A | 02-01-2002 |
| | | AU 7567601 A | 02-01-2002 |
| | | AU 8573301 A | 02-01-2002 |
| | | WO 0197755 A2 | 27-12-2001 |
| | | WO 0197756 A2 | 27-12-2001 |
| | | WO 0197760 A1 | 27-12-2001 |
| | | WO 0197763 A2 | 27-12-2001 |
| | | WO 0197757 A2 | 27-12-2001 |
| | | EP 1292263 A2 | 19-03-2003 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO. EP 04 29 0563

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

25-06-2004

| Document brevet cité au rapport de recherche | Date de publication | Membre(s) de la famille de brevet(s) | Date de publication |
|---|---|---|---|
| WO 0197755 A | | EP 1292264 A2 | 19-03-2003 |
| | | EP 1292265 A1 | 19-03-2003 |
| | | EP 1292270 A1 | 19-03-2003 |
| | | EP 1292266 A2 | 19-03-2003 |
| | | JP 2003535876 T | 02-12-2003 |
| | | JP 2003535877 T | 02-12-2003 |
| | | JP 2003535878 T | 02-12-2003 |
| | | JP 2003535880 T | 02-12-2003 |
| | | US 2003167578 A1 | 11-09-2003 |
| | | US 2003143167 A1 | 31-07-2003 |
| | | US 2003113280 A1 | 19-06-2003 |
| | | US 2003135936 A1 | 24-07-2003 |
| EP 0855178 A | 29-07-1998 | JP 10203932 A | 04-08-1998 |
| | | CN 1203071 A | 30-12-1998 |
| | | EP 0855178 A2 | 29-07-1998 |
| | | ID 19744 A | 30-07-1998 |
| US 5804172 A | 08-09-1998 | AT 205075 T | 15-09-2001 |
| | | AU 3249293 A | 19-07-1993 |
| | | CA 2125691 A1 | 24-06-1993 |
| | | DE 69232049 D1 | 11-10-2001 |
| | | DE 69232049 T2 | 06-06-2002 |
| | | EP 0679078 A1 | 02-11-1995 |
| | | WO 9311737 A1 | 24-06-1993 |
| FR 2100198 A | 17-03-1972 | CA 946598 A1 | 07-05-1974 |
| | | DE 2132509 A1 | 05-01-1972 |
| | | FR 2100198 A5 | 17-03-1972 |
| | | GB 1304979 A | 31-01-1973 |
| | | IT 1000014 B | 30-03-1976 |
| | | JP 53043908 B | 24-11-1978 |
| | | US 3696044 A | 03-10-1972 |

EPO FORM P0460

Pour tout renseignement concernant cette annexe : voir Journal Officiel de l'Office européen des brevets, No.12/82